(19) Europäisches Patentamt
European Patent Office
Office européen des brevets

(11) **EP 1 217 379 B1**

(12) **EUROPÄISCHE PATENTSCHRIFT**

(45) Veröffentlichungstag und Bekanntmachung des Hinweises auf die Patenterteilung:
**07.04.2010 Patentblatt 2010/14**

(51) Int Cl.:
***G01N 33/84*** *(2006.01)*      ***A61M 1/14*** *(2006.01)*
***A61M 1/16*** *(2006.01)*

(21) Anmeldenummer: **01129707.4**

(22) Anmeldetag: **13.12.2001**

(54) **Dialysegerät zur Ermittlung der Ionenkonzentration des Blutes**

Dialysis apparatus for determining the ion concentration of blood

Appareile de dialyse pour la détermination de la concentration des ions dans le sang.

(84) Benannte Vertragsstaaten:
**AT BE CH CY DE DK ES FI FR GB GR IE IT LI LU MC NL PT SE TR**

(30) Priorität: **22.12.2000 DE 10064179**
               **23.03.2001 DE 10114283**

(43) Veröffentlichungstag der Anmeldung:
**26.06.2002 Patentblatt 2002/26**

(73) Patentinhaber: **Fresenius Medical Care Deutschland GmbH**
**61352 Bad Homburg v.d.H. (DE)**

(72) Erfinder:
• **Nier, Volker, Dr.**
**61191 Rosbach (DE)**

• **Krämer, Matthias, Dr.**
**61381 Friedrichsdorf (DE)**

(74) Vertreter: **Laufhütte, Dieter et al**
**Lorenz-Seidler-Gossel**
**Widenmayerstrasse 23**
**80538 München (DE)**

(56) Entgegenhaltungen:
**EP-A- 0 330 892     EP-A- 0 898 976**
**EP-A- 0 952 453     WO-A-91/06326**
**US-A- 3 934 977     US-A- 4 618 587**
**US-A- 4 724 216     US-A- 5 714 060**

## Beschreibung

Die vorliegende Erfindung betrifft ein Dialysegerät mit einem Hämodialysator und/oder Hämofilter sowie mit einem extrakorporalen Blutkreislauf, mit dem Mittel zur Zugabe von Citrat zum Blut stromaufwärts des Hämodialysators und/oder Hämofilters in Verbindung stehen.

Zahlreiche Ionen, Atome und Moleküle liegen nicht isoliert, sondern in Form verschiedenster Komplexe vor. Dies betrifft sowohl Ionen, Atome und Moleküle im Blutkreislauf eines Patienten als auch in Blut, das zum Zwecke einer bestimmten Blut- bzw. Patientenbehandlung dem Patienten entnommen und außerhalb des Körpers mit geeigneten Komplexbildnern versetzt wird.

Ein Beispiel für die Komplexierung von Ionen ist die sogenannte citratantikoagulierte Hämodialyse/Hämofiltration. Hier erfolgt eine Komplexierung von $Ca^{++}$-Ionen des Blutes im extrakorporalen Blutkreislauf mittels Citrat, um während der Behandlung, vor allem während des Blut-Membran-Kontaktes im Dialysator, die Blutgerinnung zu hemmen.

Bei den meisten Hämodialysepatienten ist heute eine Hemmung der Blutgerinnung erforderlich. Standard ist die Verwendung von unfraktioniertem Heparin, das mit einer Spritzenpumpe in den arteriellen Schenkel des extrakorporalen Schlauchsystems infundiert wird. Die Verwendung sowohl von unfraktioniertem Heparin wie auch von alternativen Antikoagulantien wie niedermolekularem Heparin oder Hirudin ist für einen Teil der Patienten sowie auch für andere extrakorporale Bluttherapien aus den folgenden Gründen problematisch:

Die Antikoagulation wirkt systemisch (d. h. nicht nur beim Membrankontakt im extrakorporalen Kreislauf, sondern im ganzen Körper), was zu einer für einige Patienten kritischen Blutungsgefährdung führt. Insbesondere im intensivmedizinischen Bereich sind 30 - 40 % der Patienten blutungsgefährdet.

Einige Patienten zeigen Unverträglichkeitsreaktionen, wie z. B. die heparininduzierte Thrombozytopenie, die eine Verwendung dieser Antikoagulantien ausschließen.

Adsorptive Therapien (z. B. Leberersatztherapie) können mit einer Verwendung z. B. von Heparin inkompatibel sein, wenn dieses die (zur Adsorption von Toxinen vorgesehenen) Bindungsstellen des Adsorbers absättigt.

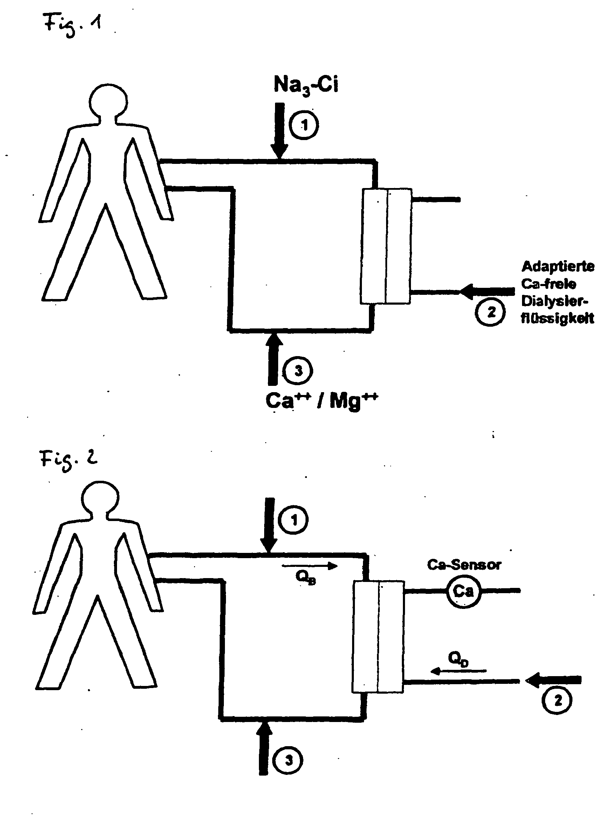

Eine Alternative, die die aufgezählten Probleme vermeidet, ist die regionale Gerinnungshemmung (nur im extrakorporalen Kreislauf, vor allem während des Blut-Membran-Kontakts) durch Citrat. Bei diesem, in Fig. 1 dargestellten Verfahren wird die Konzentration an freiem Calcium (Ca) durch Zugabe von Trinatrium-Citrat an der Stelle gemäß Pos. 1 in Fig. 1 soweit herabgesetzt, daß die Gerinnungskaskade unterbrochen wird. Zwei Citrat-Moleküle bilden dabei jeweils mit drei Ca-Ionen einen Komplex. Zur Konzentrationsreduktion trägt auch die Verwendung von Ca-freiem Dialysat gemäß Pos. 2 in Fig. 1 und der dadurch bedingte Ca-Entzug bei. Um eine Depletion des Körpers an Ca und Mg (welches ebenfalls von Citrat gebunden wird) zu vermeiden, muß eine weitere Lösung (siehe Pos. 3 in Fig. 1), welche Ca- und Mg-Ionen in adaptierter Konzentration enthält, in den venösen Schenkel des extrakorporalen Kreislaufs oder in einen separaten venösen Zugang infundiert werden.

Klinische Studien [Morita Y, Johnson RW, Dorn RE, Hall DS, "Regional anticoagulation during hemodialysis using citrate". The American Journal of the Medical Sciences (1961); Janssen MJMF et al., "Citrate compared to low molecular weight heparin anticoagulation in chronic hemodialysis patients", Kindney Int (1996) 49:806-813; Mehta RL, McDonald BR, Aguilar MM, Ward DM, "Regional citrate anticoagulation for continuous arteriovenous hemodialysis in critically ill patients". Kidney Int (1990) 38:976-981] weisen nach, daß die regionale Citrat-Antikoagulation sehr wirksam die Blutgerinnung im extrakorporalen Kreislauf verhindert. Gleichzeitig wird ein erhöhtes Blutungsrisiko für den Patienten vermieden. Die Citratdialyse gilt daher als interessante, wirkungsvolle Alternative zur herkömmlichen Heparin-Antikoagulation für jenen Teil der Patientenpopulation, bei der - wie oben beschrieben - die Verwendung von Heparin nachteilig oder klar kontraindiziert ist.

Trotz dieser klaren therapeutischen Vorteile wird die Citrat-Antikoagulation bisher nur in geringem Umfang, und nicht als automatisiertes, standardisiertes Verfahren eingesetzt. Die Gründe dafür sind:

1. Der erhöhte Aufwand: Bei der herkömmlichen Dialyse wird Standard-Dialysierflüssigkeit sowie eine geringe Menge Heparin benötigt. Bei der Citratdialyse werden üblicherweise drei Lösungen benötigt: Die Ca- und Mgfreie, in Na- und Bicarbonatgehalt adaptierte Dialysierflüssigkeit, die Trinatriumcitratlösung, und die $Ca^{++}/Mg^{++}$-Lösung.

2. Sicherheitsaspekte bei der Dosierung der $Ca^{++}/Mg^{++}$-Lösung: Wird hier falsch dosiert, kann schnell eine lebensbedrohliche Situation entstehen (Tetanie, Herzrhythmusstörungen, Herzstillstand). Eine falsche Dosierung kann sich aufgrund technischer Probleme (z. B. Ausfall der Pumpe, Lecks etc.) oder aber durch falsche Ermittlung des Ca-Bedarfs ergeben.

3. Konsequenzen einer Zufuhr von Citrat bzw. den $Ca_3$-$Ci_2$ bzw. $Mg_3$-$Ci_2$-Komplexen: Metabolische Alkalose, unphysiologische Ca-Konzentration.

Der Mehraufwand des Verfahrens kann durch geeignete technische Realisierung begrenzt werden; gewisse Mehrkosten sind bei Patienten mit Heparinunverträglichkeiten durchaus gerechtfertigt. Die Zufuhr von Citrat bzw. Citratkomplexen läßt sich durch effizienten Entzug über die Membran (Verwendung eines großflächigen Highflux-Filters, evtl. In Kombination mit postdilution-HDF) stark reduzieren. Eine geringe verbleibende Zufuhr ist wahrscheinlich tolerierbar; falls nicht, kann sie auch durch geeignete Modellierung des Dialyseprozesses und der Metabolisierung der Komplexe abgeschätzt und kompensiert werden.

Aus der WO 91/06326 ist ein citrat-antikoaguliertes Dialyseverfahren bekannt. Als Antikoagulant wird in der arteriellen Zufuhrleitung des extrakorporalen Blutkreislaufes Trinatriumcitrat zugegeben, wobei die zugegebene Menge an Trinatriumcitrat pro Zeiteinheit lediglich der Blutstromrate im extrakorporalen Kreislauf angepaßt wird. Bei einem Anstieg bzw. einer Verringerung der Blutstromrate wird die Zugaberate an Trinatriumcitrat ebenfalls erhöht bzw. erniedrigt. Die Calcium-Ionen-Konzentration des Patientenblutes wird hier nicht engmaschig überwacht, sondern in größeren Zeitabständen durch Blutproben ermittelt. Da eine engmaschige oder kontinuierliche Überwachung der CalciumKonzentration im Blut des Patienten fehlt und die Menge des zugegebenen Citrates sich lediglich nach der Blutflußrate und nicht nach dem Calciumlevel des Patienten orientiert, kann bei diesem vorbekannten Verfahren nicht sicher ausgeschlossen werden, daß sich Calcium-Werte im Blut des Patienten ergeben, die unphysiologisch sind und entsprechend lebensbedrohliche Konsequenzen für den Patienten nach sich ziehen können.

Aus US 5,714,060 A ist ein Dialysator mit einem Hämodialysator sowie mit einem extrakorporalen Blutkreislauf bekannt, mit welchem ein Beutel zur Zugabe von Kochsalzlösung zum Blut stromaufwärts des Hämodialysators sowie ein Blutsensor stromabwärts des Hämodialysators in Verbindung steht.

Zwar sind auch ionensensitive Sensoren bekannt, mittels derer die Calcium- oder Magnesium-Ionenkonzentration im Blut eines Patienten auch in kleinen Zeitintervallen ermittelt werden können. Jedoch ist die Verwendung eines blutseitigen Sensors wegen möglicher Toxizität, aufgrund von Sterilitätsanforderungen sowie aus Kostengründen nachteilig und daher unerwünscht.

Auch in anderen Anwendungsfällen als der Dialyse kann es notwendig sein, eine Konzentrationsermittlung von in Komplexen gebundenen Ionen, Atomen oder Molekülen vorzunehmen. Selbst unter der Annahme, daß ionensensitive Sensoren ohne Nachteile für den Patienten eingesetzt werden könnten, liefert deren Einsatz bei der Bestimmung der Konzentration von komplexierten Ionen oftmals keine oder nicht verwertbare Ergebnisse, da die Eigenschaften des in Komplex vorliegenden Ions eine sinnvolle Messung nicht zulassen.

Es wird ein Verfahren zur Ermittlung der Konzentration eines in einem Komplex gebundenen Ions, Atoms oder Moleküls überlegt, mittels dessen zuverlässig die Konzentration des zu bestimmenden Ions, Atoms bzw. Moleküls ermittelt werden kann. Ein solches ermittelt die Ionenkonzentration des Blutes aufgrund einer Bestimmung der Ionenkonzentration im Dialysat, wobei vor der Bestimmung der Ionenkonzentration im Dialysat die Komplexbildung des betreffenden Ions mit Citrat durch Zugabe oder Entzug einer Substanz zum Zwecke der Konzentrationsermittlung unterbunden wird.

Einem solchen Verfahren liegt somit der Gedanke zugrunde, zum Zwecke der Konzentrationsermittlung die Bildung des Komplexes mit dem Ion, Atom oder Molekül zu vermeiden oder das Ion, Atom oder Molekül aus einem derartigen Komplex abzutrennen, um dann die entsprechende Konzentrationsermittlung vornehmen zu können.

Die zugegebene Substanz kann eine Säure sein. Die Komplexbildung wird in diesem Fall durch die mit der Zugabe der Säure einhergehende pH-Wert-Änderung unterbunden. Dabei wird der Komplexbildner protoniert und damit für die Komplexbildung der zu bestimmenden Substanz gleichsam inaktiviert.

Die Komplexbildung kann dadurch unterbunden werden, daß die Zugabe des Komplexbildners unterbrochen wird oder der Komplexbildner mit einer anderen zuzugebenden Substanz einen Komplex eingeht und dabei das in seiner Konzentration zu ermittelnde Ion, Atom oder Molekül freigibt. Denkbar wäre somit die Zugabe einer Substanz, die zu dem Komplexbildner eine höhere Affinität aufweist als das zu bestimmende Ion, Atom oder Molekül. Letztere werden bei Zugabe dieser Substanz aus dem Komplex "herausgelöst" und stehen dann für eine Messung zur Verfügung.

Es ist vorgesehen, daß es sich um ein Verfahren zur Ermittlung der Ionenkonzentration des Blutes eines Patienten bei der citrat-antikoagulierten Hämodialyse und/oder Hämofiltration handelt. Dabei wird die Ionenkonzentration des Bluts aufgrund der Bestimmung der Ionenkonzentration im Dialysat ermittelt. Vor der Bestimmung der Ionenkonzentration im Dialysat wird die Komplexbildung des betreffenden Ions mit Citrat zum Zwecke der Konzentrationsermittlung unterbunden.

Dadurch läßt sich die Ionenkonzentration des Blutes eines Patienten kostengünstig und ohne Sicherheitsrisiken für den Patienten während der Behandlung überwachen.

Aufgrund der Tatsache, daß die Bestimmung der Ionenkonzentration nicht im Blut des Patienten, sondern im Dialysat erfolgt, ergibt sich der erhebliche Vorteil, daß entsprechend Sensoren zur Messung der Blut-Ionenkonzentration und die daraus resultierenden oben genannten Nachteile vermieden werden können. Ein Eingriff in den extrakorporalen Blutkreislauf ist daher zur Konzentrationsbestimmung nicht erforderlich. Entsprechendes gilt für die hier nicht notwendige wiederholte Entnahme von Blutproben.

Die Komplexbildung kann des weiteren dadurch unterbunden werden, daß die Citrat-Zugabe in den Blutkreislauf vorübergehend unterbrochen wird. Da die blutseitige Konzentration im Dialysator nicht der Konzentration im Patienten entspricht, da die Ionen, insbesondere Ca- und Mg-Ionen, durch die Komplexbildung mit Citrat gebunden werden, wird nach dieser Ausführungsform die Citrat-Infusion unterbrochen, um die tatsächliche Ionenkonzentration an Ca oder Mg auch im Dialysator zu erhalten. Die Unterbrechung der Citrat-Zugabe bzw. - Infusion kann in regelmäßigen Intervallen erfolgen. Die Infusion der Ca- und Mg-Ionen kann in diesen Zeiträumen beibehalten werden, da die durch die Unterbrechung der Citratzugabe freigesetzten Ionen weitgehend im Dialysator dem Blut entzogen werden. Ebenso kann die Infusion der Ca- und Mg-Ionen reduziert werden, um den partiellen Entzug im Dialysator gerade zu kompensieren. Diese Überwachungsmethode ist diskontinuierlich, da die Citrat-Infusion nur für kurze Intervalle unterbrochen werden kann, um eine ausreichende Antikoagulation sicherzustellen. Dies genügt jedoch, um kritische Trends rechtzeitig zu erkennen. Bei Unter- oder Überdosierung oder Totalausfall der Ca/Mg-Ionen-Ionfusion dauert es nämlich einige Zeit, bis kritische Konzentrationen im Körper erreicht werden. Diese Zeit liegt bei einigen zehn Minuten, und hängt vor allem vom Blutfluß ab.

In weiterer Ausgestaltung des Verfahrens ist vorgesehen, daß die Komplexbildung dadurch unterbunden wird, daß aus dem im Dialysat befindlichen Ion-Citrat-Komplex das Ion durch Absenkung des pH-Wertes freigesetzt wird. Die Konzentration des auf diese Weise freigesetzten Ions wird anschließend ermittelt. Ein derartiges Verfahren hat den Vorteil, daß eine Unterbrechung der Citrat-Infusion hier nicht erforderlich ist. Setzt man das betreffende Ion, vorzugsweise $Ca^{++}$ und/oder $Mg^{++}$ aus dem Ion-Citrat-Komplex frei, läßt sich das entsprechende Ion im Dialysat ohne weiteres bestimmen. Das Verfahren ist jedoch verhältnismäßig komplex, da eine zusätzliche Infusion erforderlich ist, um die Freisetzung zu bewirken. Hier muß dialysatseitig Säure zugefügt werden. Hinzu kommt, daß der Ion-Citrat-Komplex mit einem Molekulargewicht von 504 (bei Ca-Ionen) relativ groß ist, so daß eine Clearance deutlich geringer als für Elektrolyte ist. Die Messung bzw. Abschätzung der blutseitigen aus der dialysatseitigen Konzentration gestaltet sich dann entsprechend schwieriger. Bei der Einstellung eines geeigneten pH-Wertes ist darauf zu achten, daß das Ca- bzw. Mg-Ion möglichst vollständig freigesetzt wird, da eine nur teilweise Freisetzung zu geringe Blutkonzentrationswerte ergeben würde.

In weiterer Ausgestaltung des Verfahrens ist vorgesehen, daß nach Unterbrechung der Citrat-Zugabe die Messung der Ionenkonzentration im Dialysat nach Ablauf einer Zeitspanne durchgeführt wird, die sich aus einer durch Totvolumina bedingten Totzeit sowie einer zur Erreichung eines quasistationären Zustandes benötigten Zeitdauer zusammensetzt. Das Totvolumen ergibt sich aus den Volumina der Schlauchverbindung zwischen dem Infusionort für Citrat und dem Ort der Meßstelle für die entsprechende Ionen-Konzentration. Üblicherweise sind Totzeiten im Bereich von etwa 10 bis 30 s zu erwarten. Danach steigt die dialysatseitige Konzentration an. Dieser Anstieg ist nicht abrupt, sondern es ist mit Zeitkonstanten mit etwa 1 bis 2 min zu rechnen, bis ein quasistationärer Zustand erreicht wird. Darunter ist ein Zustand zu verstehen, ab dem Änderungen der Konzentrationen vernachlässigbar klein sind bzw. innerhalb einer vorgegebenen Toleranz liegen.

Gemäß einer bevorzugten Ausgestaltung des Verfahrens ist vorgesehen, daß nach Unterbrechung der Citrat-Zugabe die Messung der Ionenkonzentration im Dialysat mehrfach wiederholt wird und der Meßwert bei Erreichen eines quasistationären Zustandes ermittelt wird. Man mißt somit zu mehreren Zeitpunkten die Konzentration im Dialysat, ermittelt auf diese Weise einen Konzentrationsverlauf und kann dann beurteilen, ob die Gleichgewichtskonzentration mit ausreichender Genauigkeit erreicht ist.

In weiterer Ausgestaltung des Verfahrens ist vorgesehen, daß nach Unterbrechung der Citrat-Zugabe die Messung der Ionenkonzentration im Dialysat mehrfach wiederholt wird und der Meßwert durch Extrapolation der erhaltenen Ionenkonzentration im Dialysat ermittelt wird. Bei dieser Methode extrapoliert man somit den Gleichgewichtswert aus dem über ein ausreichendes Zeitintervall gemessenen Anstiegsverhalten der Konzentration im Dialysat. Diese Vorgehensweise weist den Vorteil auf, daß dadurch die Einstellung des Gleichgewichts nicht abgewartet werden muß. Jedoch ist hier eine Kenntnis des Zeitverhaltens des Konzentrationsanstiegs erforderlich.

Gemäß einer weiteren bevorzugten Ausgestaltung kann die Citrat-Konzentration für ein vorgegebenes Zeitintervall unterbrochen werden und der Meßwert wird durch Integration der Fläche der durch die Ionen-Konzentration im Dialysat als Funktion der Zeit definierten Antwortfunktion ermittelt. Die Citrat-Infusion kann beispielsweise für ein festes, relativ kurzes Zeitintervall, ca. 1 bis 2 min, unterbrochen werden. Durch Auswertung der Fläche unter pulsförmigen Antwortfunktion der Konzentration im Dialysat kann dann auf die Gleichgewichtskonzentration geschlossen werden.

Die Maßnahme der zeitweiligen Unterbrechung der Citrat-Infusion unterbricht selbstverständlich auch die regionale Antikoagulation im Dialysator. Jedoch ist nicht damit zu rechnen, daß sich daraus eine erhebliche Beeinträchtigung der Antikoagulation ergibt. So ist z. B. bei der sogenannten heparinfreien Dialyse, die eigentliche eine völlig antikoagulationsfreie Dialyse mit zyklischer Spülung des extrakorporalen Kreislaufes ist, die Komplikationsrate durch Blutgerinnung relativ gering (unter 5 %). Es ist daher nicht zu erwarten, daß eine zeitweise Unterbrechung der Antikoagulation, die sich dann insgesamt auf ca. 10 bis 20 % der Dialysedauer summieren dürfte, zu klinischen Problemen führt.

Wird eine Komplexbildung dadurch unterbunden bzw. aufgebrochen, daß aus dem im Dialysat befindlichen Ion-Citrat-Komplex das Ion durch Absenkung des pH-Wertes freigesetzt wird, wird vorzugsweise ein pH-Wert von 2 bis 3 eingestellt, da sich hier eine entsprechend vollständige Dissoziation des Komplexes realisieren läßt.

Die Einstellung eines pH-Wertes im Dialysat erfolgt vorzugsweise mittels einer Infusion von Säure.

Besonders vorteilhaft ist es, wenn zum Zwecke der Annäherung der Ionenkonzentration des Dialysats an die

Ionenkonzentration des Blutes der Dialysatfluß reduziert wird. Durch Reduktion des Dialysatflusses wird die dialysatseitige Ionenkonzentration der blutseitigen Ionenkonzentration angeglichen.

Es wurde theoretisch und experimentell gezeigt, daß bei einer Abnahme des Dialysatflusses bei gleichbleibendem Blutfluß die Konzentration kleinmolekularer Substanzen sich immer mehr der Blut-Plasmakonzentration annähert. Wählt man den Dialysatfluß ausreichend klein - abhängig vom Blutfluß, dem interessierenden Molekül und dem verwendeten Dialysator - ist die Dialysatkonzentration praktisch identisch zur Blutkonzentration; das Dialysat erreicht dann eine Sättigung. Im Falle von Ionen wird die Dialysatkonzentration in diesem Zustand des Fließgleichgewichts jedoch wegen des Donnan-Effekts um einige Prozent von der Blut-Plasmakonzentration abweichen, was - bei Kenntnis oder Abschätzung des Plasmaproteingehalts - rechnerisch berücksichtigt werden kann.

In weiterer Ausgestaltung des beschriebenen Verfahrens ist vorgesehen, daß die Ermittlung der Ionenkonzentration des Blutes ohne Reduzierung des Dialysatflußes durch Berechnung erfolgt. Eine derartige Vorgehensweise hat den Vorteil, daß der Dialysatfluß nicht erniedrigt werden muß und damit die Dialyseeffektivität in diesem Zeitintervall nicht reduziert wird.

Unter normalen Behandlungsbedingungen, bei denen der Dialysatfluß größer oder gleich dem Blutfluß ist, ergibt sich eine gegenüber der Blutkonzentration geringere Dialysatkonzentration. Bei Kenntnis der Dialysator-Transporteigenschaften für das betrachtete Molekül/Atom ($k_0A$), der Maschineneinstellungen (Blut- und Dialysatfluß) und des Hämatokrit läßt sich die Blut-Plasmakonzentration in guter Näherung aus der gemessenen dialysatseitigen Konzentration berechnen. Die blutseitige Konzentration berechnet sich bei Kenntnis der Clearance aus der einfachen Formel

$$C_{Bi} := (Q_D/K) * C_{Do}$$

Dabei ist $C_{Bi}$ die blutseitige Eingangskonzentration, $C_{Do}$ die gemessene dialysatseitige Ausgangskonzentration, $Q_D$ der Dialysatfluß und K die Clearance. K ist für eine in vitro-Situation mit rein diffusivem Transfer gegeben durch:

$$K := Q_B \cdot \frac{\exp\left[k0A \cdot \left(\frac{1}{Q_B} - \frac{1}{Q_D}\right)\right] - 1}{\exp\left[k0A \cdot \left(\frac{1}{Q_B} - \frac{1}{Q_D}\right)\right] - \frac{Q_B}{Q_D}}$$

Entsprechende komplexere Formalismen liegen auch für die in vivo Situation vor, ebenso für Hämodialyse mit Ultrafiltration, und für filtrative (HF) bzw. kombinierte filtrativ/diffusive (HDF) Verfahren. Dieser für jede beliebige Dialysesituation anwendbare mathematische Formalismus ist mit in vitro-und in vivo-Messungen validiert worden und steht in Form der Software "Clearance Calculation Tool CCT" von FMC in einer anwenderfreundlichen Form zur Verfügung.

Die Möglichkeit der Verringerung des Dialysatflußes ist zu bevorzugen, wenn eine genaue Messung erforderlich ist. Sie hat jedoch den Nachteil, daß für einige Minuten der Dialysatfluß erniedrigt werden muß und damit die Dialyseeffektivität in diesem Zeitintervall entsprechend reduziert ist. Sind für eine Überwachung der Ca-Ionen-Konzentration jedoch keine sehr genauen Messungen erforderlich, kann auch das Verfahren der Berechnung der Ionenkonzentration ohne Erniedrigung des Dialysatflusses angewandt werden, bei dem entsprechend keine Beeinflussung der Dialyse durch das Meßverfahren erfolgt, sieht man von der zeitweisen Unterbrechung der Antikoagulation ab.

Besonders vorteilhaft ist es, wenn die Erfassung der Ionenkonzentration im Dialysat mittels eines ionensensitiven Sensors in dem vom Dialysator abfließenden Dialysat erfolgt. Die Genauigkeit der Bestimmung der Ionenkonzentration des Blutes ist im wesentlichen durch das Sensorsystem gegeben. Da eine Fehlanzeige des Sensors in Kombination z. B. mit einer Fehldosierung ein hohes Gefahrenpotential für den Patienten darstellt, ist die Funktion des Sensors durch geeignete Prüfung oder andere Maßnahmen sicherzustellen. Eine wiederholte Funktionsprüfung des Sensors während der Dialyse kann erforderlich werden, wobei sich die Prüfintervalle an dem Zeitintervall orientieren, innerhalb dessen sich eine kritischer Zustand für den Patienten entwickeln kann (einige 10 min). Alternativ oder zusätzlich können redundante Sensorsysteme verwendet werden. Auch ist es möglich Sicherungssysteme vorzusehen, die ein charakteristisches Ausfallverhalten des Sensors sofort detektieren und zur Anzeige bringen.

In weiterer Ausgestaltung des Verfahrens ist vorgesehen, daß die ermittelte Ionenkonzentration des Blutes eines Patienten als Regelgröße dient, deren Wert durch die Stellgrößen Citrat-Zugabe und/oder Zugabe eines ionenhaltigen Substitutionsmediums beeinflußt wird. Hier ist somit ein Regelkreis mit entsprechender Regeleinheit realisierbar, durch den ein gewünschter Wert der Ca-Ionen- und/oder Mg-Ionen-Konzentration im Patientenblut eingestellt werden kann. Auf diese Weise ist ein selbständig arbeitendes Überwachungssystem für die citrat-antikoagulierten Hämodialyse und/oder Hämofiltration realisierbar, durch das stets ein physiologischer Wert der Konzentration an Ca- und/oder Mg-Ionen im Blut des Patienten eingestellt werden kann. Als Stellgrößen können die Citrat-Zugabe bzw. die Zugabe des entsprechenden ionenhaltigen Substitutionsmediums dienen. Bei der Wahl der Citrat-Zugabe als Stellgröße ist jedoch zu beachten, daß diese bestimmte Grenzen nicht unterschreiten darf, um die Ionen-Konzentration im Bereich des Dialysators gering zu halten, was erforderlich ist, um die Koagulation wirksam zu vermeiden.

Eine besonders sichere Ausführung des Verfahrens ergibt sich dadurch, daß ein Alarm ausgelöst wird, wenn die ermittelte Ionen-Konzentration im Blut des Patienten außerhalb eines zulässigen Bereiches liegt oder von einem zulässigen Wert abweicht. Ein Alarm kann ausgelöst werden, wenn eine Einzelmessung kritisch hohe oder niedrige Werte ergibt oder wenn ein kritischer Trend festgestellt wird. Zusätzlich oder alternativ zu einem Alarm können geeignete Gegenmaßnahmen eingeleitet werden, wie z. B. Bypass der Maschine, Anhalten der Infusionen, Änderung der Infusionsraten etc.

In weiterer Ausgestaltung des Verfahrens ist vorgesehen, daß die Ionenkonzentration im blutseitigen Kompartiment des Dialysators ohne Unterbrechung der Citrat-Zufuhr bestimmt und mit einem zulässigen Grenzwert verglichen wird und in Abhängigkeit dieses Vergleiches die Citrat-Zufuhr verändert wird. Eine derartige Vorgehensweise ist sinnvoll, um zu prüfen, ob die Citrat-Zugabe ausreicht, um die Ca-Ionen-Konzentration soweit abzusenken, daß das Koagulationsrisiko auf das gewünschte Maß verringert wird. Diese ist selbstverständlich bei eingeschalteter Citrat-Zufuhr zu bestimmen, da der Überschuß des freien Ca bei Citrat-Zufuhr bestimmt werden muß. Hier ist ein regelndes System möglich, das automatisch die Citrat-Infusionsrate entsprechend der gemessenen Ca-Ionen-Konzentration variiert. Auch die erforderliche Ca-/Mg-Ionen-Substitution kann dadurch bestimmt werden.

Wie oben ausgeführt handelt es sich bei den Ionen vorzugsweise um Calcium- und/oder Magnesium-Ionen, die beide von Citrat in entsprechenden Komplexen gebunden werden. Vorzugsweise wird das Verfahren unter Ermittlung der Ca-Ionen-Konzentration im Dialysat durchgeführt.

Die vorliegende Erfindung betrifft ein Dialysegerät nach Anspruch 1.

Ein erfindungsgemäßes Dialysegerät weist demnach einen Hämodialysator und/oder Hämofilter sowie einen extrakorporalen Blutkreislauf auf, mit dem Mittel zur Zugabe von Citrat zum Blut stromaufwärts des Hämodialysators und/oder Hämofilters sowie Mittel zur Zugabe einer ionenhaltigen Substitutionslösung zum Blut stromabwärts des Hämodialysators und/oder Hämofilters in Verbindung stehen, sowie mit einer Dialysatleitung, die bezüglich der Fließrichtung des Dialysats stromabwärts des Hämodialysators und/oder Hämofilters Mittel zur Erfassung einer Ionenkonzentration im Dialysat aufweist. Die Mittel zur Erfassung der Ionenkonzentration sind vorzugsweise als einer oder mehrere ionensensitive Sensoren ausgeführt. Eine redundante Ausführung erhöht die Betriebssicherheit des Dialysegerätes und ermöglicht die rasche Erkennung von Fehlmessungen.

Eine sichere Ausführungsform des Dialysegerätes läßt sich dadurch realisieren, daß eine Prüfeinrichtung vorgesehen ist, die in Zeitintervallen oder bei Betätigung durch einen Bediener eine Funktionsüberprüfung des oder der Sensoren vornimmt.

Mit der Dialysatleitung können Mittel zur Zugabe einer Substanz in Verbindung stehen, durch die der pH-Wert des Dialysats veränderbar ist. Auf diese Weise läßt sich das zu ermittelnde Ion nach dem Freisetzen aus dem Komplex durch pH-Wertänderung in seiner Konzentration ermitteln.

Die Mittel zur Zugabe der Substanz können derart angeordnet sein, daß die Zugabe bezüglich der Fließrichtung des Dialysats stromabwärts des Dialysators erfolgt.

Gemäß einer weiteren Ausgestaltung der vorliegenden Erfindung sind Mittel vorgesehen, durch die der Dialysatfluß vorübergehend verringerbar ist. Durch die Verringerung des Dialysatflusses läßt sich die Ionenkonzentration im Blut besonders genau aus der Ionenkonzentration im Dialysat berechnen.

Es kann eine Steuereinheit vorgesehen sein, die in Zeitintervallen oder bei Betätigung durch den Bediener die Mittel zur Zugabe von Citrat zum Blut derart ansteuert, daß die Zugabe vorübergehend unterbrochen wird, und die nach Beginn der Unterbrechung der Citrat-Zugabe den durch die Mittel zur Erfassung einer Ionenkonzentration im Dialysat ermittelten Konzentrationswert kontinuierlich oder in Zeitabständen aufzeichnet.

Die Steuereinheit kann derart ausgestaltet sein, daß diese den Meßwert der Ca$^{++}$-Ionenkonzentration nach einem Verfahren gemäß der Ansprüche 7 bis 10 ermittelt.

Gemäß einer weiteren bevorzugten Ausgestaltung der vorliegenden Erfindung ist eine Regeleinheit vorgesehen, die mit dem ionensensitiven Sensor sowie mit den Mitteln zur Zugabe von Citrat und/oder mit den Mitteln zur Zugabe einer ionenhaltigen Substitutionslösung verbunden ist, und die in Abhängigkeit des Vergleiches zwischen einem Sollwert oder Sollwertbereich und dem ermittelten Istwert der Ionenkonzentration eine Erhöhung oder der Zugabemenge an Citrat und/oder an ionenhaltiger Substitutionslösung veranlaßt. Auf diese Weise läßt sich die Konzentration im Blut des Patienten auf einen gewünschten Wert bzw. in einem gewünschten Intervall einregeln.

Die Regeleinheit und/oder die Mittel zur Zugabe von Citrat können derart ausgestaltet sein, daß die Konzentration an Citrat nicht unter einen Grenzwert absenkbar ist. Auf diese Weise wird verhindert, daß die Citrat-Konzentration nicht unter einen bestimmten Wert fällt, der für die wirksame Verhinderung der Koagulation erforderlich ist.

Es kann eine Alarmeinrichtung vorgesehen sein, die bei Ermittlung einer kritischen Einzelmessung der Ionenkonzentration oder bei Ermittlung eines kritischen Trends von Einzelmessungen einen Alarm auslöst. Der Bediener wird somit beispielsweise akustisch und/oder optisch auf einen derartigen kritischen Zustand hingewiesen.

Weitere Einzelheiten und Vorteile der vorliegenden Erfindung werden anhand eines in der Zeichnung dargestellten Ausführungsbeispiels näher erläutert. Es zeigen:

Fig. 1: eine schematische Darstellung eines Hämodialyseverfahrens mit Citrat-Antikoagulation mit verwendeten Lösungen und Position der Zuführung,

Fig. 2: eine schematische Darstellung gemäß Fig. 1 mit zusätzlicher Darstellung eines Ca-Ionen-Sensors in der vom Dialysator abführenden Dialysatleitung,

Fig. 3: eine Darstellung der Angleichung der dialysatseitigen Konzentration an die blutseitige Konzentration einer NaCl-Lösung bei Reduzierung des Dialysatflusses (in vitro-Experiment),

Fig. 4: eine Darstellung der Simulation des zu erwartenden Konzentrationsverlaufs im Dialysat bei zeitweiliger Unterbrechung der Citrat-Infusion und

Fig. 5: eine schematische Darstellung eines Hämodialyseverfahrens mit Citrat-Antikoagulation; Ermittlung der Ca-Ionen-Konzentration durch Dissoziation der Ca-Citrat-Komplexe im Dialysat.

Fig. 1 zeigt in schematischer Darstellung ein Hämodialyseverfahren mit Citrat-Antikoagulation. Durch die Zugabe von Trinatriumcitrat (siehe Pos. 1) in dem vom Patienten zum Dialysator führenden Teil des extrakorporalen Kreislaufs wird die Konzentration an freien Ca-Ionen soweit herabgesetzt, daß die Gerinnungskaskade unterbrochen wird. Es entsteht ein Tricalcium-Bicitratkomplex. Eine weitere Konzentrationsreduktion von Ca-Ionen im Blut wird dadurch herbeigeführt, daß Cafreies Dialysat (siehe Pos. 2) verwendet wird, so daß sich ein entsprechend hoher Gradient der Ca-Ionen-Konzentration über die Membran ergibt. Um eine unzulässige Depletion von Calcium und auch Magnesium, welches ebenfalls von Citrat gebunden wird, im Körper des Patienten zu vermeiden, wird eine Substitutionslösung (siehe Pos. 3), die Ca- und Mg-Ionen in adaptierter der Konzentration enthält, in den venösen Schenkel des extrakorporalen Kreislaufs infundiert. Die entsprechende Substitutionslösung kann selbstverständlich auch in einen separaten venösen Zugang des Patienten infundiert werden.

Zur Durchführung des Verfahrens wird die Komplexbildung des Ca-Citrat-Komplexes beispielsweise dadurch unterbunden, daß die Zugabe von Citrat vorübergehend unterbrochen wird. Entsprechend steigt die Ca-Konzentration an und es kann mit dem in Fig. 2 ersichtlichen Ca-Sensor dialysatseitig die Ca-Ionen-Konzentration gemessen werden. Die Größen QD und QB kennzeichnen den Dialysat- bzw. Blutfluß. Eine Bestimmung der Ca-Ionen-Konzentration im Patientenblut ist dadurch möglich, daß der Dialysatfluß soweit reduziert wird, daß die dialysatseitige Ca-Ionen-Konzentration der blutseitigen Ca-Ionen-Konzentration angeglichen wird. Ohne Änderung des behandlungsüblichen Blut- und Dialysatflusses kann die blutseitige Konzentration aus der gemessenen dialysatseitigen Konzentration auch mittels der bekannten oben genannten Beziehungen zwischen der blutseitigen Eingangskonzentration der Ca-Ionen, der gemessenen dialysatseitigen Ausgangskonzentration der Ca-Ionen, dem Dialysatfluß sowie der Clearance bestimmt werden.

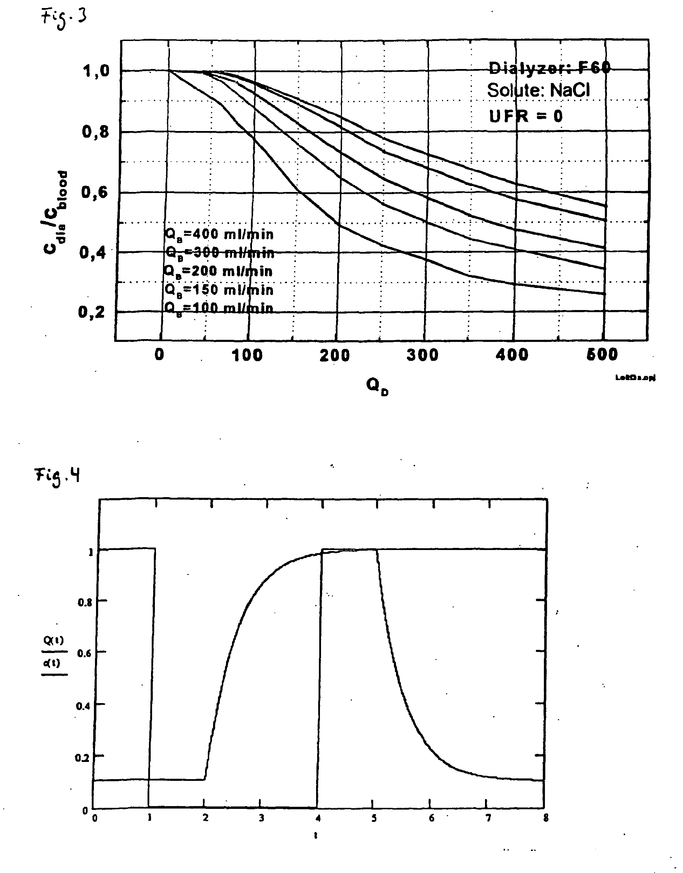

Fig. 3 zeigt die Angleichung der dialysatseitigen Konzentration einer NaCl-Lösung an die blutseitige Konzentration bei Reduzierung des Dialysatflusses. Wählt man den Dialysatfluß ausreichend klein - abhängig vom Blutfluß, dem betreffenden Molekül und dem verwendeten Dialysator - entspricht die Dialysatkonzentration praktisch der Blutkonzentration. Das Dialysat erreicht dann eine Sättigung. Auf diese Weise läßt sich aus der gemessenen Konzentration im Dialysat mit guter Genauigkeit die Konzentration im Blut bestimmen.

Fig. 4 zeigt die Antwortfunktion der Ca-Ionen-Konzentration im Dialysat bei sprunghafter Unterbrechung der Citrat-Infusion. Die dargestellte Rechteckfunktion gibt den Zeitverlauf der Citrat-Infusion wieder. Wie aus Fig. 4 ersichtlich, ist die Citrat-Infusion im Zeitintervall $t = 1$ bis $t = 4$ unterbrochen. Die andere dargestellte Kurve gibt den dialysatseitigen Konzentrationsverlauf an Ca-Ionen wieder. Die Ordinaten beider Figuren sowie die Abszisse haben beliebige Einheiten, da es hier auf Absolutwerte nicht ankommt. Nach dem Abschalten des Infusionsflusses ($t = 1$) wird zunächst am Ort des Sensors noch die niedrige Ca-Ionen-Konzentration bestehenbleiben. Grund ist, daß das Volumen der Schlauchverbindungen zwischen Infusionsort und dem Ort des Sensors als Totvolumen wirkt. Üblich sind Totzeiten im Bereich von etwa 10 bis 30 s. Wie aus Fig. 4 weiter ersichtlich, steigt anschließend die dialysatseitige Ca-Ionen-Konzentration an. Wegen der Vermischung von Blut und Dialysat jeweils mit hoher und niedriger Citrat-Konzentration vor allem im Dialysat wird der Anstieg nicht scharf sein, sondern die Konzentration wird sich an einen Gleichgewichtswert annähern. Der Anstieg ist in Fig. 4 vereinfachend als Exponentialfunktion dargestellt. Üblicherweise ist hiermit Zeitkonstanten von etwa 1 bis 2 min zu rechnen. Danach ist ein quasistationärer Zustand erreicht, so daß die hier ermittelte Konzentration mit guter Genauigkeit als Meßwert dienen kann. Nach Wiedereinschalten der Citrat-Infusion (t = 4) wird zunächst wieder die Totzeit wirksam. Anschließend sinkt die Ca-Ionen-Konzentration ab, um sich schließlich dem niedrigen Gleichgewichtswert bei laufender Citrat-Infusion anzunähern.

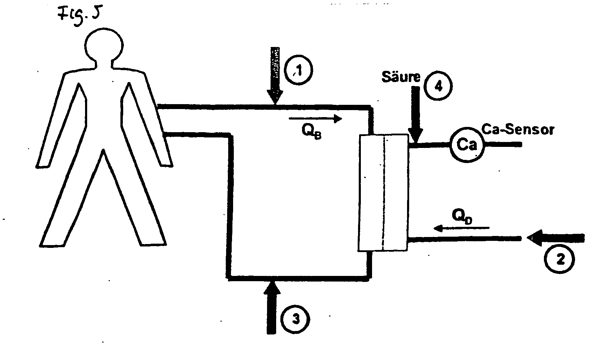

Fig. 5 zeigt eine Verfahrensvariante, bei der die Unterbrechung der Citrat-Infusion nicht erforderlich ist. Hier wird die Ca-Ionen-Konzentration dadurch bestimmt, daß aus dem Ca-Citrat-Komplex das Ca-Ion wieder freigesetzt wird. Dies geschieht durch Zufuhr einer Säure (siehe Pos. 4). Hierdurch wird der pH-Wert vorzugsweise auf einem Bereich von 2 bis 3 reduziert, was zu einer Dissoziation des Komplexes führt und die Ca-Ionen entsprechend freisetzt. Vorteil dieser Verfahrensvariante ist es, daß eine Unterbrechung der Antikoagulation nicht erfolgt, da die Citrat-Zugabe nicht unterbrochen wird.

Das beschriebene Verfahren ermöglicht es, den eigentlichen physiologisch relevanten und kritischen Parameter der Ca-Ionen- bzw. Mg-Ionen-Konzentration des Patientenblutes während der gesamten Therapie zu überwachen, so daß zu jedem Zeitpunkt der Behandlung sichergestellt ist, daß ein die Patientengesundheit gefährdender Zustand ausgeschlossen ist. Voraussetzung ist selbstverständlich, daß die Ermittlung des Ca- bzw. Mg-Ionen-Wertes zuverlässig erfolgt. Hier kann durch eine redundante Sensorausführung oder durch Sensoren, die Selbsttests durchführen eine gute Zuverlässigkeit erreicht werden. Neben dem Einsatz von Sensoren sind selbstverständlich auch andere Methoden zur Ermittlung der Ionen-Konzentration einsetzbar.

Während vorbekannte Überwachungssysteme im allgemeinen nur einzelne technische Komponenten betreffen, wie z. B. die Überwachung einer Pumpe mit separaten oder integrierten Überwachungssystemen, z. B. Tropfenzählern, die die Überwachung einer Infusion gestatten, schließt die Überwachung der Ionenkonzentration des Patienten als den eigentlichen physiologisch relevanten kritischen Parameter die gesamte Therapie ein und berücksichtigt damit jeden Teilaspekt des Verfahrens.

## Patentansprüche

1. Dialysegerät mit einem Hämodialysator und/oder Hämofilter sowie mit einem extrakorporalen Blutkreislauf, mit dem Mittel zur Zugabe von Citrat zum Blut stromaufwärts des Hämodialysators und/oder Hämofilters in Verbindung stehen,
   **gekennzeichnet durch**
   eine Dialysatleitung, die bezüglich der Fließrichtung des Dialysats stromabwärts des Hämodialysators und/oder Hämofilters Mittel zur Erfassung einer Ionenkonzentration im Dialysat aufweist, sowie Mittel zur Zugabe einer ionenhaltigen Substitutionslösung zum Blut, welche stromabwärts des Hämodialysators und/oder Hämofilters mit dem extrakorporalen Blutkreislauf in Verbindung gebracht werden können.

2. Dialysegerät nach Anspruch 1, **dadurch gekennzeichnet, daß** die Mittel zur Erfassung der Ionenkonzentration in Form eines oder mehrerer ionensensitiver Sensoren ausgeführt sind.

3. Dialysegerät nach Anspruch 2, **dadurch gekennzeichnet, daß** eine Prüfeinrichtung vorgesehen ist, die in Zeitintervallen oder bei Betätigung durch einen Bediener eine Funktionsüberprüfung des oder der Sensoren vornimmt.

4. Dialysegerät nach einem der Ansprüche 1 bis 3, **dadurch gekennzeichnet, daß** mit der Dialysatleitung Mittel zur Zugabe einer Substanz in Verbindung stehen, durch die der pH-Wert des Dialysats veränderbar ist.

5. Dialysegerät nach Anspruch 4, **dadurch gekennzeichnet, daß** die Mittel derart angeordnet sind, daß die Zugabe bezüglich der Fließrichtung des Dialysats stromabwärts des Dialysators erfolgt.

6. Dialysegerät nach einem der Ansprüche 1 bis 5, **dadurch gekennzeichnet, daß** Mittel vorgesehen sind, durch die der Dialysatfluß vorübergehend verringerbar ist.

7. Dialysegerät nach einem der Ansprüche 1 bis 6, **dadurch gekennzeichnet, daß** eine Steuereinheit vorgesehen ist, die in Zeitintervallen oder bei Betätigung durch den Bediener die Mittel zur Zugabe von Citrat zum Blut derart ansteuert, daß die Zugabe vorübergehend unterbrochen wird, und die nach Beginn der Unterbrechung der Citrat-Zugabe den durch die Mittel zur Erfassung einer Ionenkonzentration im Dialysat ermittelten Konzentrationswert kontinuierlich oder in Zeitabständen aufzeichnet.

8. Dialysegerät nach einem der Ansprüche 1 bis 7, **dadurch gekennzeichnet, daß** eine Regeleinheit vorgesehen ist, die mit dem Mittel zur Erfassung einer Ionenkonzentration im Dialysat sowie mit den Mitteln zur Zugabe von Citrat und/oder mit den Mitteln zur Zugabe einer ionenhaltigen Substitutionslösung verbunden ist, und die in Abhängigkeit des Vergleiches zwischen einem Sollwert oder Sollwertbereich und dem ermittelten Istwert der Ionenkonzentration eine Erhöhung oder Erniedrigung der Zugabemenge an Citrat und/oder an ionenhaltiger Substitutionslösung veranlaßt.

9. Dialysegerät nach Anspruch 8, **dadurch gekennzeichnet, daß** die Regeleinheit und/oder die Mittel zur Zugabe von Citrat derart ausgestaltet sind, daß die Konzentration an Citrat nicht unter einen Grenzwert absenkbar ist.

10. Dialysegerät nach einem der Ansprüche 1 bis 9, **dadurch gekennzeichnet, daß** eine Alarmeinrichtung vorgesehen ist, die bei Ermittlung einer kritischen Einzelmessung der Ionenkonzentration oder bei Ermittlung eines kritischen Trends von Einzelmessungen einen Alarm auslöst.

**Claims**

1. A dialyser having a haemo-dialyser and/or a haemo-filter and having an extra-corporal blood circulation with which means for the adding of citrate to the blood are connected upstream of the haemo-dialyser and/or of the haemo-filter, **characterised by**
   a dialysate line which has means for the detection of an ion concentration in the dialysate downstream of the haemo-dialyser and/or of the haemo-filter with respect to the direction of flow of the dialysate; and
   means for the adding of a substitution solution containing ions to the blood which can be brought into connection with the extra-corporal blood circulation downstream of the haemo-dialyser and/or of the haemo-filter.

2. A dialyser in accordance with claim 1, **characterised in that** the means for the detection of the ion concentration are designed in the form of one or more ion-sensitive sensors.

3. A dialyser in accordance with claim 2, **characterised in that** a test device is provided which performs a function check of the sensor(s) at time intervals or on actuation by an operator.

4. A dialyser in accordance with one of the claims 1 to 3, **characterised in that** means for adding a substance are connected to the dialysis line by which the pH of the dialysate can be changed.

5. A dialyser in accordance with claim 4, **characterised in that** the means are disposed such that the addition takes place downstream of the dialyser with respect to the direction of flow of the dialysate.

6. A dialyser in accordance with one of the claims 1 to 5, **characterised in that** means are provided by which the dialysate flow can be reduced temporarily.

7. A dialyser in accordance with one of the claims 1 to 6, **characterised in that** a control unit is provided which controls the means for adding citrate to the blood at time intervals or on actuation by the operator such that the addition is temporarily interrupted and which records the concentration value determined by the means for detecting an ion concentration in the dialysate after the start of the interruption of the citrate addition continuously or at time intervals.

8. A dialyser in accordance with one of the claims 1 to 7, **characterised in that** a regulating unit is provided which is connected to the means for detecting an ion concentration in the dialysate and to the means for adding citrate and/or to the means for adding a substitution solution containing ions and which initiates an increase or a lowering of the addition amount of citrate and/or of substitution solution containing ions in dependence on the comparison between a nominal value or a nominal value range and the determined actual value of the ion concentration.

9. A dialyser in accordance with claim 8, **characterised in that** the regulating unit and/or the means for adding citrate are designed such that the concentration of citrate cannot be lowered below a threshold value.

10. A dialyser in accordance with one of the claims 1 to 9, **characterised in that** an alarm unit is provided which triggers an alarm on determination of a critical individual measurement of the ion concentration or on determination of a critical trend of individual measurements.

**Revendications**

1. Appareil de dialyse avec un hémodialyseur et/ou un hémofiltre ainsi qu'avec une circulation sanguine extracorporelle, avec lequel des moyens d'addition de citrate au sang se trouvent en relation en amont de l'hémodialyseur et/ou de l'hémofiltre,
**caractérisé par**
un conduit de produit dialysé, qui présente des moyens de détection d'une concentration des ions dans le produit dialysé par rapport au sens d'écoulement du produit dialysé en aval de l'hémodialyseur et/ou de l'hémofiltre, ainsi que des moyens d'addition d'une solution ionique de substitution au sang, qui peuvent être mis en relation avec la circulation sanguine extracorporelle en aval de l'hémodialyseur et/ou de l'hémofiltre.

2. Appareil de dialyse selon la revendication 1, **caractérisé en ce que** les moyens de détection de la concentration des ions sont construits sous la forme d'un ou plusieurs capteurs ionosensibles.

3. Appareil de dialyse selon la revendication 2, **caractérisé en ce qu'**un dispositif de contrôle est prévu, qui effectue un contrôle du fonctionnement du ou des capteurs par intervalles de temps ou en cas d'actionnement par un utilisateur.

4. Appareil de dialyse selon une quelconque des revendications 1 à 3, **caractérisé en ce que** des moyens d'addition d'une substance, par laquelle la valeur de pH du produit dialysé est modifiable, se trouvent en relation avec le conduit de produit dialysé.

5. Appareil de dialyse selon la revendication 4, **caractérisé en ce que** les moyens sont disposés de manière à ce que l'addition par rapport au sens d'écoulement du produit dialysé s'effectue en aval du dialyseur.

6. Appareil de dialyse selon une quelconque des revendications 1 à 5, **caractérisé en ce que** des moyens sont prévus, par lesquels l'écoulement de produit dialysé est réductible provisoirement.

7. Appareil de dialyse selon une quelconque des revendications 1 à 6, **caractérisé en ce qu'**une unité de commande est prévue, qui amorce les moyens d'addition de citrate au sang par intervalles de temps ou en cas d'actionnement par l'utilisateur de manière à ce que l'addition soit interrompue provisoirement, et qui, après le début de l'interruption de l'addition de citrate, enregistre de manière continue ou par intervalles de temps la valeur de concentration déterminée par les moyens de détection d'une concentration des ions dans le produit dialysé.

8. Appareil de dialyse selon une quelconque des revendications 1 à 7, **caractérisé en ce qu'**une unité de régulation est prévue, qui est reliée avec le moyen de détection d'une concentration des ions dans le produit dialysé ainsi qu'avec les moyens d'addition de citrate et/ou avec les moyens d'addition d'une solution ionique de substitution, et qui, en fonction de la comparaison entre une valeur de consigne ou une plage de valeurs de consigne et la valeur réelle de la concentration des ions, entraîne une augmentation ou une réduction du volume d'addition de citrate et/ou de solution ionique de substitution.

9. Appareil de dialyse selon la revendication 8, **caractérisé en ce que** l'unité de régulation et/ou les moyens d'addition de citrate sont coordonnés de manière à ce que la concentration de citrate ne soit pas abaissable au-dessous d'une valeur limite.

10. Appareil de dialyse selon une quelconque des revendications 1 à 9, **caractérisé en ce qu'**un dispositif d'alarme est prévu, qui déclenche une alarme en cas de détermination d'une mesure individuelle critique de la concentration des ions ou en cas de détermination d'une tendance critique de mesures individuelles.

Fig. 1

Na$_3$-Ci

① 

Adaptierte
Ca-freie
Dialysierflüssigkeit

②

③

Ca$^{++}$ / Mg$^{++}$

Fig. 2

①

Q$_B$

Ca-Sensor

Ca

Q$_D$

②

③

**Fig. 3**

**Fig. 4**

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9106326 A
- US 5714060 A

### In der Beschreibung aufgeführte Nicht-Patentliteratur

- **Morita Y ; Johnson RW ; Dorn RE ; Hall DS.** Regional anticoagulation during hemodialysis using citrate. *The American Journal of the Medical Sciences,* 1961
- **Janssen MJMF et al.** Citrate compared to low molecular weight heparin anticoagulation in chronic hemodialysis patients. *Kindney Int,* 1996, vol. 49, 806-813
- **Mehta RL ; McDonald BR ; Aguilar MM ; Ward DM.** Regional citrate anticoagulation for continuous arteriovenous hemodialysis in critically ill patients. *Kidney Int,* 1990, vol. 38, 976-981